# FASCICULE DE BREVET EUROPEEN

(11) **EP 1 361 854 B1**
(45) Date de publication et mention de la délivrance du brevet: **11.04.2007**
(21) Numéro de dépôt: 02704872.7
(22) Date de dépôt: 21.02.2002
(51) Int. Cl.: A61K 8/49, A61K 8/34, A61Q 19/00

(54) **SOLUTION TOPIQUE COMPRENANT UNE ISOFLAVONE AGLYCONE**
TOPISCHE LÖSUNG ENTHALTEND EIN ISOFLAVON-AGLYKON
TOPICAL SOLUTION COMPRISING A ISOFLAVONE AGLYCONE

(30) Priorité: 21.02.2001 FR 0102319
(43) Date de publication de la demande: 19.11.2003
(73) Titulaire: Laboratoires Expanscience, 92400 Courbevoie (FR)
(72) Inventeur: MSIKA, Philippe, F-75018 Paris (FR); PICCIRILLI, Antoine, F-28230 Epernon (FR); PICCARDI, Nathalie, 38120 Saint-Egreve (FR); BROUTIN, Nicole, F-28800 Alluyes (FR)
(74) Mandataire: Ahner, Francis
(86) Numéro de dépôt international: PCT/FR2002/000650
(87) Numéro de publication internationale: WO 2002/066001

(56) Documents cités:
- EP-A- 0 774 249
- EP-A- 1 104 672
- WO-A-99/38509
- WO-A-99/47118
- FR-A- 2 769 502
- US-A- 6 004 558
- US-A- 6 019 992
- US-A- 6 060 070
- US-B1- 6 180 662
- PATENT ABSTRACTS OF JAPAN vol. 009, no. 192 (C-296), 8 août 1985 (1985-08-08) & JP 60 061513 A (SANSHIYOU SEIYAKU KK), 9 avril 1985 (1985-04-09)

## Description

La présente invention concerne un procédé de préparation de compositions destinées à un usage externe topique comprenant une solution vraie contenant au moins une isoflavone aglycone choisie parmi la génistéine, la daidzéine, la glycitéine, et leurs mélanges et un solvant choisi parmi les PEG, PPG, et leurs mélanges. L'invention a également pour objet l'utilisation de ces compositions en tant qu'agents photo-protecteurs de la peau, avantageusement en tant qu'agents ayant une activité anti-érythémale, anti-oxydante, anti-radicalaire.

Les composés de type flavonoïdes tels que les flavones, flavanones, flavonols, aurones, chalcones, naturels ou synthétiques, sont de puissants antioxydants et présentent souvent de fortes activités inhibitrices des enzymes en général. Ces propriétés expliquent l'utilisation de ces molécules actives dans les domaines cosmétique, pharmaceutique et nutritionnel.

En particulier, la génistéine est connue comme agent préventif contre les problèmes de peau et les cancers de peau induits par les rayons UV (WO 97/46208). La génistéine peut ainsi être administrée par voie topique pour prévenir et traiter les inflammations et les irritations de la peau telles que les érythèmes.

La demanderesse a découvert de manière surprenante que les activités photo-protectrices, notamment les activités anti-érythémale, anti-oxydante, anti-radicalaire, d'un composé tel que la génistéine augmentent de manière significative lorsque ledit composé est préalablement solubilisé dans un solvant approprié, tel que les polyéthylène glycols, les polypropylène glycols, et leurs mélanges.

Le polyéthylène glycol est un solvant connu des isoflavones de soja. Des formulations alimentaires sous forme de micro/nano émulsions, contenant des micelles de très faible diamètre, peuvent ainsi être obtenues en mélangeant un isoflavonoïde avec le polyéthylène glycol 400 (WO 99/38509). Toutefois, les micro/nano émulsions n'étant pas homogènes, la quantité de principe actif contenue dans ce type d'émulsions est difficile à déterminer et la préparation de compositions contenant ces micro/nano émulsions est difficile à mettre en oeuvre.

Or, il a été découvert, de manière surprenante selon l'invention, qu'il était possible d'utiliser le polyéthylène glycol en association avec un composé tel que la génistéine pour former une solution vraie, dépourvue des inconvénients cités ci-dessus, dans une composition cosmétique ou dans un médicament apte à être utilisé par voie externe topique et destinée à agir en tant qu'agent photo-protecteur (de surface et cellulaire) de la peau, et plus particulièrement en tant qu'agent anti-érythémal, anti-oxydant, anti-radicalaire.

De plus, les polyéthylène glycols, les polypropylène glycols, s'avèrent être des composés cosmétiquement acceptables, non agressifs pour la peau, non toxiques, hypoallergéniques et qui procurent l'avantage, pour le formulateur, de faciliter la formation d'émulsions stables tout en assurant via leur pouvoir solvant, la solubilité des principes actifs de la composition.

Ainsi, la solubilisation d'un composé tel qu'un flavonoïde préalablement à son incorporation dans une composition cosmétique ou pharmaceutique procure non seulement l'avantage d'augmenter la pénétration et la biodisponibilité dudit composé en vue de potentialiser et d'améliorer ses activités biologiques, telles que son activité photo-protectrice sur la peau, mais aussi de faciliter la préparation et la mise en oeuvre d'une telle composition.

La présente invention a ainsi pour objet un procédé de préparation de compositions destinées à un usage externe topique comprenant au moins une solution vraie contenant au moins un composé (a) dissous dans un solvant (b), ledit solvant (b) étant choisi dans le groupe constitué par les polyéthylène glycols, les polypropylène glycols, et leurs mélanges et ledit composé (a) étant choisi parmi la génistéine, la daidzéine, la glycitéine, et leurs mélanges.

Par le terme de "solution vraie", on entend au sens de la présente invention, toute solution homogène, claire ou limpide qui ne soit pas une micro ou une nano émulsion, qui ne contienne pas de micelles et dont la concentration en principe actif demeure appréciable et précisément quantifiable par dosage chromatographique.

Selon la présente invention, le flavonoïde est une isoflavone aglycone. Les isoflavones utilisables selon la présente invention sont obtenues par synthèse chimique ou sont des substances naturelles extraites de produits naturels, notamment à partir de végétaux. On distingue les formes aglycones des isoflavones et les formes glycosylées de ces dernières. Ces diverses formes sont illustrées par les formules suivantes.
Formes aglycones, de formule : 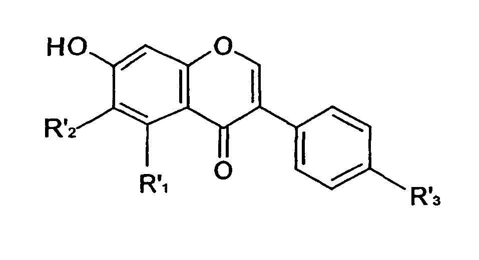 dans laquelle R'₁ représente un atome d'hydrogène ou un groupe hydroxy, R'₂ représente un atome d'hydrogène ou un groupe méthoxy et R'₃ représente un groupe hydroxy.

Avantageusement, selon la présente invention, R'₁, R'₂ et R'₃ représentent :

| **R'₁** | **R'₂** | **R'₃** | **Nom du composé** |
|---|---|---|---|
| H | H | OH | Daidzéine |
| OH | H | OH | Génistéine |
| H | OCH₃ | OH | Glycitéine |

Formes glycosylées, de formule : 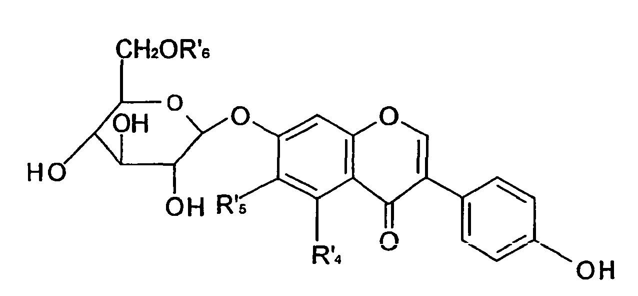 dans laquelle R'₄ représente un atome d'hydrogène ou un groupe hydroxy, R'₅ représente un atome d'hydrogène ou un groupe méthoxy et R'₆ représente un atome d'hydrogène.
Avantageusement, selon la présente invention R'₄, R'₅ et R'₆ représentent :

| **R'₄** | **R'₅** | **R'₆** | **Nom du composé** |
|---|---|---|---|
| H | H | H | Daidzine |
| OH | H | H | Génistine |
| H | OCH₃ | H | Glycitine |

Les formes glycosylées des isoflavones sont les plus abondantes dans la nature. Cependant, les isoflavones aglycones présentent des activités biologiques nettement supérieures à leurs homologues glycosylés. Tel est le cas des isoflavones naturelles telles que la génistéine (1), la daidzéine ou la glycitéine.

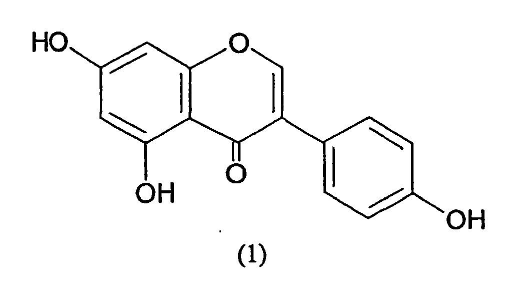

Selon la présente invention, l'isoflavone est une isoflavone aglycone, choisie dans le groupe constitué par la génistéine, la daidzéine et la glycitéine. De façon encore plus avantageuse, le flavonoïde (a) est la génistéine. En particulier, la génistéine utilisable selon la présente invention est un produit d'origine végétale, titrant 85 à 90 % en poids.

Le solvant (b) est choisi dans le groupe constitué par les polyéthylène glycols et les polypropylène glycols, avantageusement de faibles poids moléculaires. Par le terme de polyéthylène glycols et de polypropylène glycols "de faibles poids moléculaires", on entend au sens de la présente invention, les polyéthylène glycols et les polypropylène glycols ayant un poids moléculaire moyen inférieur ou égal à 600, avantageusement compris entre 200 et 600. De façon encore plus avantageuse, le solvant (b) est le polyéthylène glycol 300 (PEG300).

De façon encore plus avantageuse, le composé (a) est présent à une concentration comprise entre 0,1 et 1,2 % en poids par rapport au poids total de la composition destinée à un usage externe topique, et en particulier le composé (a) est présent à une concentration comprise entre 0,1 et 12 % en poids par rapport au poids total de la solution vraie telle que définie ci-dessus, constituée par le composé (a) dissous dans le solvant (b). Avantageusement, le solvant (b) est présent à une concentration comprise entre 0,5 et 15 % en poids par rapport au poids total de la composition destinée à un usage externe topique.

Les compositions selon la présente invention peuvent par ailleurs contenir des excipients cosmétiquement ou pharmaceutiquement acceptables, ainsi que des additifs cosmétiques conventionnels, notamment des filtres organiques ou minéraux UVB/UVA, des antioxydants, des anti-radicalaires, des protecteurs de la cellule irradiée, des actifs anti-âge connus de l'homme du métier (rétinoïdes, vitamines, avocadofuranes, insaponifiables, etc.).

Les compositions selon la présente invention peuvent en particulier se présenter sous forme de lotion aqueuse ou hydroalcoolique, monophasique ou polyphasique, de gel monophasique ou polyphasique, d'émulsion, de crème, de dispersion vésiculaire, de mousse ou de spray.

La présente invention a pour objet un procédé de préparation de ces compositions, caractérisé en ce qu'il comprend l'étape de solubilisation du composé (a) dans le solvant (b), à température ambiante, sous agitation, pendant une durée comprise entre 10 et 120 minutes, avantageusement pendant 30 minutes.

La présente invention a aussi pour objet une composition destinée à un usage externe topique susceptible d'être obtenue par le procédé selon l'invention.

La présente invention a également pour objet une composition selon l'invention pour son utilisation comme médicament.

La présente invention a également pour objet l'utilisation des compositions décrites ci-dessus pour la préparation d'un médicament destiné à agir comme agent photo-protecteur de la peau, en particulier comme agent anti-oxydant, comme agent anti-radicalaire destiné à prévenir et/ou à réduire les érythèmes, en particulier les érythèmes actiniques induits par des irradiations UVA et/ou UVB.

Avantageusement, les compositions de la présente invention sont appliquées directement sur la peau avant et/ou pendant et/ou après exposition au soleil, et avantageusement après l'apparition d'un érythème sur la peau.

Enfin, la présente invention a pour objet l'utilisation d'une solution vraie comprenant au moins un composé (a) dissous dans un solvant (b), ledit composé (a) étant une isoflavone aglycone choisie parmi la génistéine, la daidzéine, la glycitéine et leurs mélanges et ledit solvant (b) étant choisi dans le groupe constitué par les polyéthylène glycols, les polypropylène glycols, et leurs mélanges, pour la préparation de compositions destinées à un usage topique externe.

Les exemples suivants sont donnés à titre non limitatif et illustrent la présente invention.

### Exemple de réalisation de l'invention

*Mode opératoire :* Une quantité de flavonoïde (a) engagée sous forme de poudre est pesée précisément dans un bécher de 250 ml. On procède ensuite à l'ajout d'une quantité précise d'excipient (b). Le mélange est ensuite vigoureusement agité (agitation mécanique) à température ambiante et pendant 30 minutes. La solution est alors réchauffée à 50°C afin d'être clarifiée par simple passage sur un filtre Büchner. La solution vraie ainsi formée est ensuite incorporée à la composition.

### Exemples de formulations cosmétiques

### Exemple 1 : Génistéine dans PEG 300

| | % en poids |
|---|---|
| Eau | QSP 100 |
| Squalane | 7,0 |
| Pétrolatum | 3,0 |
| Glycérine | 3,0 |
| Néopentanoate d'isodécyle | 3,0 |
| Tetraéthylhexanoate de pentaérythrityle | 3,0 |
| Cyclométhicone | 2,0 |
| Alcool cétéarylique | 2,0 |
| Myristate de myristyle | 1,0 |
| Laureth-23 | 1,0 |
| Cire d'abeille | 1,0 |
| Gomme de Sclerotium | 1,0 |
| Glucoside de cétéaryle | 0,6 |
| Palmitate de cétyle | 0,1 |
| Cocoglycérides | 0,1 |
| PEG-300 | 4.0 |
| Génistéine | 0,4 |
| Conservateurs | QS |

### Exemple 2 : Génistéine dans PEG 300 :

| | % en poids |
|---|---|
| Eau | QSP 100 |
| Squalane | 7,0 |
| Pétrolatum | 3,0 |
| Glycérine | 3,0 |
| Néopentanoate d'isodécyle | 3,0 |
| Tetraéthylhexanoate de pentaérythrityle | 3,0 |
| Cyclométhicone | 2,0 |
| Alcool cétéarylique | 2,0 |
| Myristate de myristyle | 1,0 |
| Laureth-23 | 1,0 |
| Cire d'abeille | 1,0 |
| Gomme de Sclerotium | 1,0 |
| Glucoside de cétéaryle | 0,6 |
| Palmitate de cétyle | 0,1 |
| Cocoglycérides | 0,1 |
| **PEG-300** | **1,8** |
| **Génistéine** | **0,2** |
| Conservateurs | QS |

### Essais de solubilité de la génistéine dans les polyéthylène glycols ou leurs homologues éthoxylés

### Mode opératoire :

La génistéine mise en oeuvre dans les tests de solubilité est un produit d'origine végétale, titrant 85 à 90 % en poids.
La quantité de génistéine engagée sous forme de poudre est pesée précisément dans un bécher de 250 ml. On procède ensuite à l'ajout d'une quantité précise d'excipient. Le mélange est ensuite vigoureusement agité (agitation mécanique) à température ambiante et pendant 30 minutes. Le produit est ensuite directement dosé selon le protocole décrit ci-dessous.

### Protocole de dosage :

Le dosage de la génistéine est réalisé par chromatographie liquide haute performance, en phase inverse (phase stationnaire en C18 - phase mobile acide acétique/acétonitrile) et détection par spectrophotométrie U.V. (au maximum d'absorption du composé). L'étalonnage externe utilise une substance de référence pure à 99 %.
Cette technique a été validée du point de vue de sa spécificité (vis-à-vis de la génistéine et autres flavones, mais aussi vis-à-vis des PEG de dilution utilisés), de sa répétabilité et de sa linéarité (de 80 % à 120 % de la valeur théorique).

### Résultats :

Le Tableau 1 rassemble divers essais de solubilité de la génistéine dans les polyéthylène glycols (PEGs) ou leurs homologues éthoxylés. La quantité de génistéine dosée par rapport à la quantité de génistéine engagée dans le solvant permet de conclure sur la solubilité de la génistéine dans ce solvant.

**Tableau 1 :**

| | | **Génistéine engagée** | **Génistéine dosée** | |
|---|---|---|---|---|
| **Excipient** | **Type** | **(% en poids dans la solution)** | **(% en poids dans la solution)** | **Solubilité** |
| PEG 200 | Polyéthylène glycol | 12.0 | 10.9 | élevée |
| PEG 300 | Polyéthylène glycol | 12.0 | 11.2 | élevée |
| PEG 400 | Polyéthylène glycol | 12.0 | 10.1 | élevée |
| PEG 600 | Polyéthylène glycol | 12.0 | 10.7 | élevée |

Les PEGs sont donc de bons solvants de la génistéine. Aussi, le pouvoir solvant des PEGs est d'autant plus marqué que leur poids moléculaire est faible, puisque nous observons des solubilités plus élevées en présence des PEGs 200 et 300. Un maximum de solubilité est toutefois observé en présence des PEGs 300.

Le tableau 2 qui rassemble des essais de solubilité de la génistéine, à diverses concentrations dans le PEG 300, corrobore ce résultat.

**Tableau 2 :**

| **Excipient** | **Type** | **Génistéine engagée (% en poids dans la solution)** | **Génistéine dosée (% en poids dans la solution)** | **Solubilité** |
|---|---|---|---|---|
| PEG 300 | Polyéthylène glycol | 8.0 | 7.6 | élevée |
| PEG 300 | Polyéthylène glycol | 10.0 | 9.3 | élevée |
| PEG 300 | Polyéthylène glycol | 12.0 | 11.2 | élevée |

La génistéine peut donc être incorporée sous forme soluble, dans le PEG 300, à une concentration proche de 12 % en poids par rapport au poids total de la solution.

### Influence de la solubilisation et de la dose de génistéine sur la réduction de l'érythème actinique

### 1- PROTOCOLE

La mise en évidence de la réduction de l'érythème actinique induit par une irradiation UVA + B avec les compositions selon la présente invention a été réalisée chez des volontaires sains à l'aide d'un test conduit en double insu avec un placebo. Le placebo (P1) et les produits actifs (P2), (P3) et (P4) ont ainsi été utilisés pour effectuer ce test.

Le principe actif, la forme galénique ainsi que la solubilisation de ces différents produits sont reportés dans le tableau suivant :

**Tableau 3 :**

| Référence test | Produit | % final de génistéine dans la crème | Forme galénique | Solubilisation |
|---|---|---|---|---|
| GE 100 Lot 135-05-0 G (P1) | Excipient | - | - | - |
| GE 100 Lot 135-05-0 A (P2) | Génistéine 85% | 1,2 | Poudre dispersée | Faible |
| GE 100 Lot 135-05-0 B (P3) | Génistéine 85% | 1,2 | Solution à 12 % de génistéine dans du PEG 300 | Elevée |
| GE 100 Lot 135-05-0 D (P4) | Génistéine 85% | 0,6 | Solution à 12 % de génistéine dans du PEG 300 | Elevée |

Plusieurs étapes ont été suivies :
- **Etape préliminaire :** Evaluation de la Dose Erythèmale Minimale individuelle (DEMᵢ).
- **Première étape :** Application des produits P1 à P4 sur une surface de 35 cm². La quantité appliquée est de 4 mg/cm² exactement. Les zones d'application des produits sont modifiées par rotation d'un volontaire à un autre.
- **Deuxième étape :** Irradiation des zones traitées et d'une zone non traitée (zone témoin) quinze minutes après l'application des produits, en positionnant la DEMᵢ au troisième trou.
- **Troisième étape :** Lectures visuelles de l'érythème 6 et 24 heures après l'irradiation.

### a.) CHOIX DES SUJETS

10 sujets par étude appariée sont choisis dans un panel de volontaires où ils sont sélectionnés selon les critères d'inclusion suivants :
- Cheveux brun, chocolat, blond, ou brun roux
- Présence ou absence d'éphélides
- Age compris entre 20 et 50 ans
- Sexe indifférent
- Absence de réaction anormale à la lumière

### b.) IRRADIATIONS

L'érythème actinique est déclenché par des irradiations effectuées avec une source xénon 150 watts équipée d'un filtre dichroïque (réduction des radiations IR) et d'un filtre Schott WG 320, de 1 mm d'épaisseur. Ceci correspond à un soleil de midi, le 21 juin, à 35° de latitude Nord.
Les irradiations sont pratiquées sur 6 zones (circulaires, diamètre: 0,8 cm) disposées en rangées, recevant des doses en progression géométrique (x 1,25).
Au préalable, avec le même appareil, la DEM individuelle de chaque sujet a été déterminée sur le haut du dos.
Chacune des rangées en expérience reçoit 0,64 - 0,8 - 1 - 1,25 - 1,56 - 1,95 DEM.

Les irradiations sont effectuées symétriquement de part et d'autre de la colonne vertébrale.
Les temps d'irradiation des zones traitées et de la zone non traitée sont déterminés de manière à placer la DEMᵢ au troisième trou.

### c.) DOSIMETRIE

Les doses UV administrées sont calculées à l'aide d'un dosimètre ROBERTSON-BERGER calibré et équipé avec une tête de lecture UVB. Les résultats sont exprimés directement en valeurs lues sur le dosimètre et exprimés en DEM (1 DEM = 25 mJ/cm²).

### d.) OBSERVATIONS

Les observations visuelles des érythèmes actiniques sont effectuées à la 6ème heure et à la 24ème heure. On évalue les érythèmes selon la classification internationale de T (traces) à +++, en notant la présence éventuelle d'un oedème.
On effectue une quantification numérique correspondant au degré d'érythème selon le tableau suivant :

**Tableau 4 :**

| **Codification de la lecture** | **Codification des résultats** |
|---|---|
| 0 | 0 |
| T | 1 |
| ± | 2 |
| + | 3 |
| + | 4 |
| +⁺ | 5 |
| ++⁻ | 6 |
| ++ | 7 |
| ++⁺ | 8 |
| +++ | 9 |

### 2 - RESULTATS

Les résultats de la réduction érythémale visuelle des produits P1, P2, P3 et P4 6 heures et 24 heures après une irradiation à la dose IDEM sont donnés dans le tableau 5. La réduction de l'érythème actinique est exprimée en pourcentage.

**Tableau 5 :**

| | **P1** | **P2** | **P3** | **P4** |
|---|---|---|---|---|
| **6 heures** | 24,32 % | 40,54% | 81,08 % | 75,68 % |
| **24 heures** | 10,00 % | 32,50 % | 77,50 % | 67,50 % |

Les résultats de la réduction érythémale visuelle des produits P1, P2, P3, P4 6 heures et 24 heures après une irradiation à la dose 1, 56 x DEM sont donnés dans le tableau suivant :

**Tableau 6 :**

| | **P1** | **P2** | **P3** | **P4** |
|---|---|---|---|---|
| **6 heures** | 11,32 % | 22,64 % | 66,04 % | 45,28 % |
| **24 heures** | 3,17% | 15,87% | 71,43% | 46,03% |

Le détail de l'évaluation visuelle des érythèmes actiniques en peau non traitée et en peau traitée, pour différentes doses significatives, à 1DEM et à 1,56 DEM, est donnée respectivement dans les tableaux 7 et 8 :

**Tableau 7 : Lectures visuelles des érythèmes 6 heures et 24 heures après l'irradiation, pour les produits P1 à P4 pour la dose IDEM**

| | **N°** | **CODE** | **MED** | **P1** | **P2** | **P3** | **P4** | **TEMOIN** |
|---|---|---|---|---|---|---|---|---|
| **6 Heures** | 1 | G004 | 1,95 | 2 | 1 | 0 | 1 | 4 |
| **6 Heures** | 2 | A121 | 1,56 | 1 | 1 | 1 | 1 | 1 |
| **6 Heures** | 3 | B002 | 3,05 | 4 | 2 | 1 | 1 | 4 |
| **6 Heures** | 4 | A117 | 2,44 | 3 | 3 | 1 | 1 | 4 |
| **6 Heures** | 5 | F067 | 2,44 | 2 | 4 | 1 | 1 | 4 |
| **6 Heures** | 6 | K014 | 1,95 | 4 | 2 | 1 | 1 | 4 |
| **6 Heures** | 7 | 1087 | 1,56 | 1 | 1 | 0 | 0 | 4 |
| **6 Heures** | 8 | L017 | 2,44 | 2 | 2 | 0 | 1 | 4 |
| **6 Heures** | 9 | G026 | 1,56 | 4 | 4 | 1 | 1 | 4 |
| **6 Heures** | 10 | H117 | 1,95 | 5 | 2 | 1 | 1 | 4 |
| **24 Heures** | 1 | G004 | 1,95 | 4 | 3 | 0 | 1 | 4 |
| **24 Heures** | 2 | A121 | 1,56 | 4 | 4 | 1 | 2 | 4 |
| **24 Heures** | 3 | B002 | 3,05 | 4 | 3 | 1 | 2 | 4 |
| **24 Heures** | 4 | A117 | 2,44 | 2 | 2 | 1 | 1 | 4 |
| **24 Heures** | 5 | F067 | 2,44 | 3 | 2 | 1 | 1 | 4 |
| **24 Heures** | 6 | K014 | 1,95 | 4 | 2 | 1 | 1 | 4 |
| **24 Heures** | 7 | 1087 | 1,56 | 3 | 3 | 1 | 1 | 4 |
| **24 Heures** | 8 | L017 | 2,44 | 4 | 3 | 1 | 2 | 4 |
| **24 Heures** | 9 | G026 | 1,56 | 4 | 3 | 1 | 1 | 4 |
| **24 Heures** | 10 | H117 | 1,95 | 4 | 2 | 1 | 1 | 4 |
| | | | | | | | | |
| **Moyenne 6h** | | | **2,09** | **2,80** | **2,20** | **0,70** | **0,90** | **3,70** |
| Student/T 6h | | | | 0,054 | 0,003 | 0,000 | 0,000 | |
| **Réduction/T** | | | | **24,32%** | **40,54%** | **81,08%** | **75,68%** | |
| Student/P1 6h | | | | | 0,217 | 0,000 | 0,001 | |
| **Réduction/P1** | | | | | **21,43%** | **75,00%** | **67,86%** | |
| | | | | | | | | |
| **Moyenne 24h** | | | **2,09** | **3,60** | **2,70** | **0,90** | **1,30** | **4,00** |
| Student/T 24h | | | | 0,104 | 0,000 | 0,000 | 0,000 | |
| **Réduction/T** | | | | **10,00%** | **32,50%** | **77,50%** | **67,50%** | |
| Student/P1 24h | | | | | 0,004 | 0,000 | 0,000 | |
| Réduction/P1 | | | | | 25,00% | 75,00% | 63,89% | |
| | | | | | | | | |
| **Moyenne** | | | **2,09** | **3,20** | **2,45** | **0,80** | **1,10** | **3,85** |
| Student/T | | | | 0,012 | 0,000 | 0,000 | 0,000 | |
| **Réduction/T** | | | | **16,88%** | **36,36%** | **79,22%** | **71,43%** | |
| Student/P1 | | | | | 0,007 | 0,000 | 0,000 | |
| **Réduction/P1** | | | | | **23,44%** | **75,00%** | **65,63%** | |

**Tableau 8 : Lectures visuelles des érythèmes 6 heures et 24 heures après l'irradiation, pour les produits P1 à P4 pour la dose 1,56xDEM**

| | **N°** | **CODE** | **MED** | **P1** | **P2** | **P3** | **P4** | **TEMOIN** |
|---|---|---|---|---|---|---|---|---|
| **6 Heures** | 1 | G004 | 1,95 | 5 | 4 | 0 | 4 | 7 |
| **6 Heures** | 2 | A121 | 1,56 | 1 | 1 | 1 | 1 | 1 |
| **6 Heures** | 3 | 8002 | 3,05 | 7 | 5 | 4 | 4 | 7 |
| **6 Heures** | 4 | A117 | 2,44 | 5 | 5 | 3 | 4 | 5 |
| **6 Heures** | 5 | F067 | 2,44 | 5 | 5 | 4 | 4 | 7 |
| **6 Heures** | 6 | K014 | 1,95 | 4 | 2 | 1 | 1 | 4 |
| **6 Heures** | 7 | 1087 | 1,56 | 5 | 5 | 1 | 4 | 5 |
| **6 Heures** | 8 | L017 | 2,44 | 5 | 5 | 2 | 4 | 7 |
| **6 Heures** | 9 | G026 | 1,56 | 5 | 5 | 1 | 1 | 5 |
| **6 Heures** | 10 | H117 | 1,95 | 5 | 4 | 1 | 2 | 5 |
| **24 Heures** | 1 | G004 | 1,95 | 7 | 7 | 1 | 4 | 7 |
| **24 Heures** | 2 | A121 | 1,56 | 5 | 5 | 1 | 3 | 5 |
| **24 Heures** | 3 | B002 | 3,05 | 8 | 5 | 4 | 5 | 8 |
| **24 Heures** | 4 | A117 | 2,44 | 5 | 5 | 1 | 4 | 7 |
| **24 Heures** | 5 | F067 | 2,44 | 7 | 5 | 3 | 4 | 7 |
| **24 Heures** | 6 | K014 | 1,95 | 5 | 4 | 2 | 1 | 5 |
| **24 Heures** | 7 | 1087 | 1,56 | 7 | 7 | 2 | 4 | 7 |
| **24 Heures** | 8 | L017 | 2,44 | 5 | 4 | 2 | 4 | 5 |
| **24 Heures** | 9 | G026 | 1,56 | 7 | 7 | 1 | 1 | 7 |
| **24 Heures** | 10 | H117 | 1,95 | 5 | 4 | 1 | 4 | 5 |
| | | | | | | | | |
| **Moyenne 6h** | | | **2,09** | **4,70** | **4,10** | **1,80** | **2,90** | **5,30** |
| Student/T 6h | | | | 0,081 | 0,009 | 0,000 | 0,000 | |
| **Réduction/T** | | | | **11,32%** | **22,64%** | **66,04%** | **45,28%** | |
| Student/P1 6h | | | | | 0,051 | 0,000 | 0,002 | |
| **Réduction/P1** | | | | | **12,77%** | **61,70%** | **38,30%** | |
| | | | | | | | | |
| **Moyenne 24h** | | | **2,09** | **6,10** | **5,30** | **1,80** | **3,40** | **6,30** |
| Student/T 24h | | | | 0,343 | 0,015 | 0,000 | 0,000 | |
| **Réduction/T** | | | | **3,17%** | **15,87%** | **71,43%** | **46,03%** | |
| Student/P1 24h | | | | | 0,037 | 0,000 | 0,000 | |
| Réduction/P1 | | | | | 13,11% | 70,49% | 44,26% | |
| | | | | | | | | |
| **Moyenne** | | | **2,09** | **5,40** | **4,70** | **1,80** | **3,15** | **5,80** |
| Student/T | | | | 0,042 | 0,000 | 0,000 | 0,000 | |
| **Réduction/T** | | | | **6,90%** | **18,97%** | **68,97%** | **45,69%** | |
| Student/P1 | | | | | 0,003 | 0,000 | 0,000 | |
| **Réduction/P1** | | | | | **12,96%** | **66,67%** | **41,67%** | |

### 3 - INTERPRETATION DES RESULTATS

### • Relation entre P1 (placebo) et T (témoin peau non traitée) :

D'après les résultats de la lecture visuelle, on constate que 6 heures et 24 heures après l'irradiation des zones traitées et non traitées, les résultats ne sont pas significatifs pour l'excipient P1 (Student p > 0,05). Cependant, lorsque l'on cumule les résultats à 6 heures et à 24 heures, et donc que l'on augmente le nombre d'observations (20), on constate que les résultats sont significatifs (Student p < 0,05). On peut donc affirmer que l'excipient contient des actifs permettant une légère réduction érythémale.
On effectue par la suite une comparaison des produits étudiés P2 à P4, par rapport à l'excipient P1 et par rapport au témoin T.

### • Significativité des résultats en comparant les produits P1 à P4 au témoin peau non traitée (test de Student) :

### Produit P1

Pour le produit P1, les résultats ne sont pas significatifs aux doses 1DEM et 1,56×DEM, 6 heures et 24 heures après l'irradiation (Student p > 0,05).

### Produits P2 à P4

Pour les produits P2 à P4, on constate que 6 heures et 24 heures après l'irradiation, aux doses IDEM et 1,56xDEM, les résultats sont significatifs (Student p < 0,05).

### • Significativité des résultats en comparant les produits P2 à P4 à l'excipient P1 (test de Student) :

### Produit P2

Pour le produit P2, on constate que, aux doses de 1DEM et de 1,56×DEM, les résultats ne sont pas significatifs 6 heures après l'irradiation des zones traitées et non traitées (Student p > 0,05). Les résultats sont cependant significatifs pour les deux doses, 24 heures après l'irradiation et lorsque l'on totalise les résultats à 6 et à 24 heures (Student p < 0,05).

### Produits P3 et P4

Pour les produits P3 et P4, on constate que, aux doses de IDEM et de 1,56×DEM, les résultats sont significatifs'6 heures et 24 heures après l'irradiation (Student p < 0,05).

### • Classement décroissant des produits P2 à P4 par rapport à la réduction érythèmale visuelle à la dose IDEM

- 24 heures après l'irradiation : P3 - P4 - P2
- En cumulant les résultats pour les observations à 6 heures et à 24 heures après l'irradiation : P3 - P4 - P2

### 4 - CONCLUSIONS

Deux conclusions majeures peuvent être émises concernant cette étude :
- La solubilisation de la génistéine est un facteur essentiel à son activité anti-érythémale. En effet, l'activité anti-érythémale de la génistéine est augmentée lorsque cette dernière est préalablement solubilisée dans du PEG 300.
- L'activité anti-érythémale de la génistéine est dépendante de la dose utilisée. En effet, le produit P3 (1,2 % de génistéine) est plus efficace que le produit P4 (0,6 % de génistéine) 6 et 24 heures après l'irradiation.

## Revendications

1. Procédé de préparation d'une composition destinée à un usage externe topique comprenant une solution vraie contenant au moins un composé (a) dans un solvant (b), ledit solvant (b) étant choisi dans le groupe constitué par les polyéthylène glycols, les polypropylène glycols, et leurs mélanges, et ledit composé (a) étant une isoflavone aglycone choisie dans le groupe constitué par la génistéine, la daidzéine, la glycitéine, et leurs mélanges, **caractérisé en ce qu'**il comprend la solubilisation du composé (a) dans le solvant (b) de manière à former une solution vraie, puis l'incorporation de la solution vraie dans la composition.

2. Procédé selon la revendication 1, **caractérisé en ce que** la solubilisation du composé (a) dans le solvant (b) est réalisée à température ambiante, sous agitation, pendant une durée comprise entre 10 et 120 minutes, avantageusement pendant 30 minutes.

3. Procédé selon la revendication 1 ou 2, **caractérisé en ce que** le solvant (b) est choisi dans le groupe constitué par les polyéthylène glycols et les polypropylène glycols de faibles poids moléculaires.

4. Procédé selon la revendication 3, **caractérisé en ce que** le solvant (b) a un poids moléculaire compris entre 200 et 600.

5. Procédé selon la revendication 4, **caractérisé en ce que** le solvant (b) est le polyéthylène glycol 300.

6. Procédé selon l'une quelconque des revendications précédentes, **caractérisé en ce que** le composé (a) est présent à une concentration comprise entre 0,1 et 1,2 % en poids par rapport au poids total de la composition.

7. Procédé selon la revendication 6, **caractérisé en ce que** le composé (a) est présent à une concentration comprise entre 0,1 et 12 % en poids par rapport au poids total de la solution vraie telle que définie dans la revendication 1.

8. Composition destinée à un usage externe topique susceptible d'être obtenue par le procédé selon l'une quelconque des revendications 1 à 7.

9. Composition selon la revendication 8 pour son utilisation comme médicament.

10. Composition selon la revendication 8, **caractérisée en ce qu'**il s'agit d'une composition cosmétique, destinée à être appliquée directement sur la peau avant et/ou pendant et/ou après exposition au soleil, avantageusement après l'apparition d'un érythème sur la peau.

11. Utilisation d'une composition selon la revendication 8 pour la préparation d'un médicament destiné à agir comme agent photo-protecteur de la peau, avantageusement comme agent anti-oxydant, comme agent anti-radicalaire, ou destiné à réduire et/ou à prévenir les érythèmes, avantageusement les érythèmes actiniques induits par des irradiations UVA et/ou UVB.

12. Utilisation d'une solution vraie comprenant au moins un composé (a) dissous dans un solvant (b), ledit composé (a) étant une isoflavone aglycone choisie dans le groupe constitué par la génistéine, la daidzéine, la glycitéine, et leurs mélanges, et ledit solvant (b) étant choisi dans le groupe constitué par les polyéthylène glycols, les polypropylène glycols, et leurs mélanges, pour la préparation de compositions destinées à un usage topique externe.

## Claims

1. Method for preparing a composition intended for external topical use, comprising a true solution containing at least one compound (a) in a solvent (b), the said solvent (b) being chosen from the group consisting of polyethylene glycols, polypropylene glycols and mixtures thereof, and the said compound (a) being an aglycone isoflavone chosen from the group consisting of genistein, daidzein, glycitein and mixtures thereof, **characterized in that** it comprises solubilizing the compound (a) in the solvent (b) so as to form a true solution, and then incorporating the true solution into the composition.

2. Method according to Claim 1, **characterized in that** the solubilization of the compound (a) in the solvent (b) is carried out at room temperature, with stirring, for a period of between 10 and 120 minutes, advantageously for 30 minutes.

3. Method according to Claim 1 or 2, **characterized in that** the solvent (b) is chosen from the group consisting of polyethylene glycols and polypropylene glycols having low molecular weights.

4. Method according to Claim 3, **characterized in that** the solvent (b) has a molecular weight of between 200 and 600.

5. Method according to Claim 4, **characterized in that** the solvent (b) is polyethylene glycol 300.

6. Method according to any one of the preceding claims, **characterized in that** the compound (a) is present at a concentration of between 0.1 and 1.2% by weight relative to the total weight of the composition.

7. Method according to Claim 6, **characterized in that** the compound (a) is present at a concentration of between 0.1 and 12% by weight relative to the total weight of the true solution as defined in Claim 1.

8. Composition intended for external topical use, which can be obtained by the method according to any one of Claims 1 to 7.

9. Composition according to Claim 8, for its use as a medicament.

10. Composition according to Claim 8, **characterized in that** it is a cosmetic composition intended to be applied directly to the skin before and/or during and/or after exposure to sunlight, advantageously after the appearance of an erythema on the skin.

11. Use of a composition according to Claim 8, for the preparation of a medicament intended to act as a sun protection agent for the skin, advantageously as an antioxidant, as an anti-free-radical agent, or intended to reduce and/or prevent erythemas, advantageously actinic erythemas induced by UVA and/or UVB radiation.

12. Use of a true solution comprising at least one compound (a) dissolved in a solvent (b), the said compound (a) being an aglycone isoflavone chosen from the group consisting of genistein, daidzein, glycitein and mixtures thereof, and the said solvent (b) being chosen from the group consisting of polyethylene glycols, polypropylene glycols and mixtures thereof, for the preparation of compositions intended for external topical use.

## Patentansprüche

1. Verfahren zur Herstellung einer Zusammensetzung, die für eine äußerliche topische Anwendung bestimmt ist, umfassend eine echte Lösung, welche wenigstens eine Verbindung (a) in einem Lösemittel (b) enthält, wobei das Lösemittel (b) in der aus den Polyethylenglycolen, den Polypropylenglycolen und deren Mischungen bestehenden Gruppe ausgewählt wird und wobei die Verbindung (a) ein Isoflavonaglycon ist, welches in der aus Genistein, Daidzein, Glycitein und deren Mischungen bestehenden Gruppe ausgewählt wird, **dadurch gekennzeichnet, dass** es die Solubilisierung der Verbindung (a) in dem Lösemittel (b) derart, dass eine echte Lösung gebildet wird, dann das Einarbeiten der echten Lösung in die Zusammensetzung umfasst.

2. Verfahren nach Anspruch 1, **dadurch gekennzeichnet, dass** die Solubilisierung der Verbindung (a) in dem Lösemittel (b) bei Umgebungstemperatur unter Bewegung während einer Dauer zwischen 10 und 120 min, vorteilhafterweise während 30 min ausgeführt wird.

3. Verfahren nach Anspruch 1 oder 2, **dadurch gekennzeichnet, dass** das Lösemittel (b) in der aus den Polyethylenglycolen und den Polypropylenglycolen mit niedrigen Molekulargewichten bestehenden Gruppe ausgewählt wird.

4. Verfahren nach Anspruch 3, **dadurch gekennzeichnet, dass** das Lösemittel (b) ein Molekulargewicht zwischen 200 und 600 eingeschlossen hat.

5. Verfahren nach Anspruch 4, **dadurch gekennzeichnet, dass** das Lösemittel (b) Polyethylenglycol 300 ist.

6. Verfahren nach einem der vorangegangenen Ansprüche, **dadurch gekennzeichnet, dass** die Verbindung (a) in einer Konzentration zwischen 0,1 und 1,2 Gew.-% eingeschlossen bezogen auf das Gesamtgewicht der Zusammensetzung vorhanden ist.

7. Verfahren nach Anspruch 6, **dadurch gekennzeichnet, dass** die Verbindung (a) in einer Konzentration zwischen 0,1 und 12 Gew.-% eingeschlossen bezogen auf das Gesamtgewicht der echten Lösung, wie in Anspruch 1 definiert, vorhanden ist.

8. Zusammensetzung, welche für eine äußerliche topische Anwendung bestimmt ist, welche durch das Verfahren nach einem der Ansprüche 1 bis 7 erhalten werden kann.

9. Zusammensetzung nach Anspruch 8 für deren Verwendung als Arzneimittel.

10. Zusammensetzung nach Anspruch 8, **dadurch gekennzeichnet, dass** es sich um eine kosmetische Zusammensetzung handelt, die dazu bestimmt ist, direkt auf die Haut vor und/oder während und/oder nach der Exposition gegenüber der Sonne, vorteilhafterweise nach dem Auftreten eines Erythems auf der Haut, aufgetragen zu werden.

11. Verwendung einer Zusammensetzung nach Anspruch 8 für die Herstellung eines Arzneimittels, welches dazu bestimmt ist, als Lichtschutzmittel für die Haut, vorteilhafterweise als Antioxidationsmittel, als gegen Radikale wirkendes Mittel zu wirken, oder dazu bestimmt ist, die Erytheme, vorteilhafterweise die aktinischen Erytheme, die durch UVA- und/oder UVB-Strahlung induziert werden, zu verringern und/oder zu verhüten.

12. Verwendung einer echten Lösung, welche wenigstens eine Verbindung (a) gelöst in einem Lösemittel (b) umfasst, wobei die Verbindung (a) ein Isoflavonaglycon ist, welches aus der aus Genistein, Daidzein, Glycitein und deren Mischungen bestehenden Gruppe ausgewählt wird und das Lösemittel (b) in der aus den Polyethylenglycolen, den Polypropylenglycolen und deren Mischungen bestehenden Gruppe ausgewählt wird, für die Herstellung von Zusammensetzungen, die für eine topische äußerliche Anwendung bestimmt sind.
